**Europäisches Patentamt**

**European Patent Office**

**Office européen des brevets**

(11) Veröffentlichungsnummer: **0 117 905**
**B1**

## (12) EUROPÄISCHE PATENTSCHRIFT

(45) Veröffentlichungstag der Patentschrift:
06.09.89

(51) Int. Cl.⁴: **A 61 K 7/16, A 61 K 7/26**

(21) Anmeldenummer: 83111676.9

(22) Anmeldetag: 22.11.83

(54) **Mundpflegemittel.**

(30) Priorität: **27.01.83 DE 3302694**

(43) Veröffentlichungstag der Anmeldung:
**12.09.84 Patentblatt 84/37**

(45) Bekanntmachung des Hinweises auf die Patenterteilung:
**06.09.89 Patentblatt 89/36**

(84) Benannte Vertragsstaaten:
**AT BE CH DE FR GB IT LI LU NL**

(56) Entgegenhaltungen:
**DE-A- 2 240 352**
**DE-B- 1 030 519**
**GB-A- 615 323**
**US-A- 4 311 602**

**DIE PHARMAZIE, Band 5, Heft 1-12, 1950, Seiten 556-557, Berlin, DE; H. KREITMAIR: "Hypericum perforatum - das Johanniskraut"**

**Die Akte enthält technische Angaben, die nach dem Eingang der Anmeldung eingereicht wurden und die nicht in dieser Patentschrift enthalten sind.**

(73) Patentinhaber: **RETHETO Filmtechnik Thellemann & Co.,
Rüdesheimer Strasse 11, D-8000 München 21 (DE)**

(72) Erfinder: **Thellemann, Horst, Rüdesheimer Strasse 11,
D-8000 München 21 (DE)**

(74) Vertreter: **Patentanwälte Grünecker, Dr. Kinkeldey, Dr.
Stockmair, Dr. Schumann, Jakob, Dr. Bezold, Meister,
Hilgers, Dr. Meyer-Plath, Maximilianstrasse 58,
D-8000 München 22 (DE)**

**Beschreibung**

Die Erfindung bezieht sich auf ein Mittel zur Mundpflege, insbesondere als Zahnpasta und Mundwasser.

Das Ziel jeder Mundhygiene besteht vorrangig in der Prophylaxe von Zahn- und Zahnfleischerkrankungen, wie Karies und Parodontose sowie in der Bekämpfung von Mundgeruch. Angesichts einer Karieshäufigkeit, die in der Bundesrepublik Deutschland mit mehr als 90% angegeben wird und einer vermuteten Parodontosehäufigkeit von 50% bis 80% der Bevölkerung, ist es als vorrangig anzusehen, prophylaktische und/oder therapeutische Methoden zu entwickeln die geeignet erscheinen, die Karies- und Parodontosehäufigkeit drastisch zu reduzieren. In den letzten 30 Jahren wurden daher eine große Anzahl neuer Stoffe und Stoffmischungen entwickelt, die als prophylaktische und/oder therapeutische Zusätze für Mundpflegemittel, insbesondere Zahnpasten und Mundwässer, geeignet erschienen. Wenn auch über den offensichtlich komplexen Mechanismus der Karies- und Parodontoseentstehung keine einheitliche Auffassung besteht, sondern bis heute noch verschiedene Theorien nebeneinander existieren, so ist doch immerhin bekannt, daß beispielsweise bei der Kariesentstehung anorganische Teile der Zahnhartsubstanzen durch Säureeinwirkung demineralisiert und/oder chelatisiert werden und die organischen Teile der Zahnhartsubstanzen durch Proteolyse abgebaut werden. Einen wesentlichen Anteil an der zahnzerstörenden Tätigkeit haben dabei Mikroorganismen, deren physiologische Aktivitäten insbesondere für die Entstehung von Karies ursächlich sind.

Im Zuge des wachsenden Verständnisses der Karies- und Parodontoseentstehung wurden den Mundhygienemitteln, also Zahnpasten und Mundwässern, besondere Wirkstoffe beigemischt, die beispielsweise antimikrobielle Wirkung zeigen oder die Mineralisierung unterstützen. Die Brauchbarkeit von Bakteriziden in Mundpflegemitteln wird jedoch bezweifelt, da die ständige Einwirkung desinfizierender Maßnahmen die physiologische Mundflora schwächen kann (H. Kliewe, Th. Lammers, Mittgsbl. Ärzteschaft Rhld.-Pfalz 1951). Im übrigen ist es bis heute nicht möglich, einen Stoff zur Verfügung zu stellen, der ein so breites Wirkungsspektrum aufweist, daß eine bakteriostatische Aktivität auch unter dauerndem Speichelfluß und in Anwesenheit von Speiseresten ausreichen würde (Th. Lammers «Mikrobielle Probleme im Bereich der Kostemtik», Hüthig, Heidelberg 1971, S. 196).

Ein guter, der kariösen Demineralisierung entgegenwirkender Einfluß wird Fluorverbindungen zugeschrieben. Eine Problematik bei der Verwendung von Fluorverbindungen in Zahnpasten besteht jedoch darin, daß einerseits zum Erzielen einer spürbaren Karieshemmung Fluor-Ionen vorliegen müssen, die mit dem Hydroxylapatit des Zahnschmelzes reagieren können. Diese Fluor-Ionen setzen sich jedoch auch mit Calcium-Ionen, die in kreidehaltigen Zahnpasten vorhanden sind, zu unlöslichem Calciumfluorid um (D. Barrie, Amer, Perfum. 75 (12) 1960). Derartige fluoridierte Zahnpasten sind jedoch unwirksam.

Zur wirksamen Bekämpfung von Karies und Parodontose wurden weiterhin eine große Anzahl von Zusätzen empfohlen, beispielsweise die Vitamine A, C, D sowie die der B-Gruppe, Myrrhentinktur und Honig, Aluminiumlactat, synthetische Gerbstoffe, Penicillin und andere Antibiotika, Kochsalz, durch dessen osmotische Wirkung eine Drainage des Zahnfleischs bewirkt wird, Azulen, das als entzündungshemmender Zusatz empfohlen wird, Borax und Borsäure, Per-Verbindungen, quartäre Ammoniumverbindungen, Äthanol, sowie Chlorophyll und Antihistaminika. Diesen Zusätzen wurden prophylaktische, konservierende oder therapeutische Effekte zugesprochen.

Für alle bisher diskutierten Stoffe gilt jedoch, daß sie jeweils nur eine der für Karies und Parodontose ursächlichen Komponenten bekämpfen. Für einen ausreichenden Schutz müssen daher mehrere dieser Stoffe in einem Mundpflegemittel verwendet werden.

Mit allen, genannten Zusätzen, deren bester prophylaktischer Wert wohl den Fluorverbindungen zukommen dürfte, ist es jedoch nicht gelungen, die oben genannte Häufigkeit von Karies und Parodontose spürbar zu reduzieren.

Der Erfindung liegt die Aufgabe zugrunde, Mittel zur Mundpflege, insbesondere Zahnpasten und Mundwässer, zur Verfügung zu stellen, mit denen mit hohem Wirkungsgrad und ohne unerwünschte Nebenwirkungen der Karies- und Prodontoseentstehung entgegengewirkt werden kann.

Diese Aufgabe wird erfindungsgemäß dadurch gelöst, daß man Mitteln zur Mundpflege, insbesondere Zahnpasten Mundwässern zusätzlich zu Johanniskrautöl Brenztraubensäure und L-Borneol und/oder Bornylacetat zugibt.

Johanniskrautöl gewinnt man als Ölauszug aus frischen Blüten des Johanniskrauts (Hypericum perforatum). Es handelt sich dabei um ein rötliches klares Öl, dessen Farbe durch einen Gehalt von mindestens 0,004% des roten Farbstoffs Hypericin bedingt ist. Im Zusammenhang mit dem erfindungsgemäßen Mundpflegemittel zeigte sich die überraschende und unerwartete Wirkung des Johanniskrautöls, sich als dünner, aber äußerst wirksam schützender Film über Zähne und Zahnfleisch zu legen. Dieser Schutzfilm hat die Eigenschaft, auch über längere Zeiträume wirksam zu bleiben, so daß die Zähne und das Zahnfleisch auch dann optimal geschützt bleiben, wenn eine Mundpflege nur ein oder zweimal pro Tag erfolgt.

Die Bildung eines Schutzfilms über die gefährdeten Zähne und Teile des Zahnfleischs aus Johanniskrautöl bewirkt, daß alle an der Karies- und Parodontoseentstehung beteiligten Schadstoffe, also alle Arten von Speiseresten, insbesondere Zuckerbestandteile, sowie Mikroorganismen ferngehalten werden. Da insbesondere kariöse Schädigungen nach heutigem Wissen auf einen exogenen Prozeß zurückzuführen sind, dessen zerstörische Wirkung durch ein Zusammenwirken von Zahnschmelz, Mundschleimhaut, Speichel sowie Speiseresten und Mikroorganismen bedingt zu sein scheint, wird mit einem filmbildenden Mittel eine optimale Prophylaxe ermöglicht.

Es konnte weiterhin gezeigt werden, daß an der Entstehung von Parodontose ursächlich beteiligten Zahnsteinablagerungen durch regelmäßige Verwendung von erfindungsgemäßen Mundpflegemitteln verhindert werden konnte, da der am Zahn und am Zahnfleisch anliegende Schutzfilm Ablagerungen unterbindet. Auf diese Weise bleibt das Zahnfleisch am Zahn anliegend.

Eine weitere Wirkung der Verwendung von Johanniskrautöl in Mundpflegemitteln ist der Abbau des insbesondere durch Mikroorganismen hervorgerufenen Zahnbelags. Der Aufbau eines neuen Zahnbelags wird durch die oben geschilderte Schutzfilmwirkung verhindert.

Durch die erfindungsgemäße Verwendung von Johanniskrautöl mit Brenztraubensäure und L-Borneol und/oder Bornylacetat in Mundpflegemitteln erreicht man somit einen synergistischen Effekt gegen alle karies- und parodontoseverursachende Komponenten, der auf den filmbildenden Wirkungsmechanismen beruht.

Die erfindungsgemäße Verwendung von Johanniskrautöl mit Brenztraubensäure und L-Borneol und/oder Bornylacetat in Mundpflegemitteln weist noch eine weitere vorteilhafte Besonderheit auf, die in Anbetracht der nach wie vor beachtlichen Nachlässigkeit der Bevölkerung bezüglich der Mundpflege bei der Karies- und Parodontosebekämpfung wesentlich ist. Dieser Vorteil liegt in der Beständigkeit des durch das Johanniskrautöl gebildeten Schutzfilms über einen langen Zeitraum. Das Einnehmen von kleineren Mahlzeiten ohne anschließendes Zähneputzen ist daher weitgehend unschädlich. Überdies erzeugt eine Mundpflege unter Verwendung eines erfindunsgemäßen Mundpflegemittels bereits ein angenehmeres Sauberkeitsgefühl im Vergleich zu bekannten Mundpflegemitteln.

Die geschilderten Vorteile und Wirkungsweisen des erfindungsgemäßen Mittels zur Mundpflege werden in optimaler Weise verwirklicht, wenn das Johanniskrautöl in einem Anteil von etwa 5 bis 15, vorzugsweise 7 Gew.%, enthalten ist. Die Zugabe des Johanniskrautöls in dieser Menge gewährleistet eine vollständige Filmbildung über den Zähnen und dem Zahnfleisch und sichert gleichzeitig eine problemlose galenische Zubereitung.

Die oben geschilderte, vorteilhafte Wirkung des Johanniskrautöls wird durch das Beimischen von Brenztraubensäure und L-Borneol und/oder Bornylacetat als weitere Bestandteile in überraschendem und unerwartet starkem Maße gesteigert.

Brenztraubensäure mit der Formel $H_3C-CO-COOH$ ist der einfachste Vertreter der $\alpha$-Ketosäuren und nimmt im Energiestoffwechsel des tierischen wie pflanzlichen Organismus eine zentrale Stellung im Citronensäurezyklus ein. Durch enzymatische Aminierung in der Leber entsteht weiterhin aus Brenztraubensäure das Alanin, ein Baustein der Proteinsynthese und im Muskelgewebe wird Brenztraubensäure zu Milchsäure reduziert. Es handelt sich somit bei der Brenztraubensäure um eine natürliche, physiologische Substanz. Es hat sich gezeigt, daß diese physiologische Substanz bei äußerlicher Anwendung eine entzündungshemmende Wirkung aufweist. Da die Brenztraubensäure eine Stoffwechselsubstanz des menschlichen Körpers ist, ist zu erwarten, daß diese Substanz nebenwirkungsfrei ist.

Ebenfalls natürlichen Ursprungs sind die Substanzen L-Borneol und Bornylacetat. Borneol hat man im Aleppo-Kiefernnadelöl, in Baldrianöl, Citronellöl, Korianderöl, Thujaöl u. a. nachweisen können. Bornylacetat ist in großen Mengen (25 bis 40%) im sibirischen Fichtennadelöl und dem Öl der Douglasfichtennadeln enthalten. Borneol und Bornylacetat weisen ebenfalls entzündungshemmende Wirkung auf. Dieser Effekt kommt bei der Verwendung dieser Substanzen in dem erfindungsgemäßen Mittel zur Mundpflege im Zusammenwirken mit dem filmbildenden Johanniskrautöl besonders gut zur Geltung, da diese entzündungshemmenden Substanzen durch die beschriebenen Eigenschaften des Johanniskrautöls flächig verteilt im gesammten Zahn- und Mundbereich langanhaltend wirksam sein können. Durch die Kombination der filmbildenden Eigenschaft des Johanniskrautöls und den entzündungshemmenden Wirkungen der Substanzen L-Borneol und Bornylacetat tritt eine kombinierte mechanisch-physiologische synergistische Wirkung ein. In diesem Fall ist die Wirkung des erfindungsgemäßen Mittels zur Mundpflege nicht nur prophylaktisch, indem durch einen Schutzfilm ein Zusammenwirken von destruktiven Substanzen mit dem Zahnschmelz oder dem Zahnfleisch verhindert wird; vielmehr hat das Zumischen von stark entzündungshemmenden Substanzen insbesondere für das Regenerieren des Zahnfleischs auch therapeutischen Wert, der durch das langfristige Einwirken noch verstärkt wird.

Die geschilderte synergistische Wirkung des erfindungsgemäßen Mittels zur Mundpflege unter Verwendung von Johanniskrautöl, Brenztraubensäure und L-Borneol und/oder Bornylacetat kommt am besten zum Tragen, wenn die Anteile der genannten Wirksubstanzen etwa so gewählt werden, daß das Verhältnis von Johanniskrautöl zu Brenztraubensäure 70 bis 1000 : 1, vorzugsweise 100 : 1, sowie das Verhältnis von Johanniskrautöl zu L-Borneol und/oder Bornylacetat 70 bis 1000 : 1, vorzugsweise 100 : 1, beträgt. Im Rahmen dieses Mischungsverhältnisses ist ein optimales Zusammenspiel der teils mechanischen und teils pyhsiologischen Wirkungsweise der verwendeten Substanzen gewährleistet.

Das erfindungsgemäße Mittel zur Mundpflege wird zweckmäßiger Weise in Form einer Zahnpasta angewandt. Das übliche Zähneputzen mit einer solchen Zahnpasta dient im vorliegenden fall jedoch lediglich der Reinigung von Essensresten, die verhältnismäßig schnell bewirkt ist. Das bisher übliche, sorgfältige und minutenlange Bürsten wird bei Verwendung einer Zahnpasta mit den erfindungsgemäßen Bestandteilen Johanniskrautöl und Brenztraubensäure und L-Borneol und/oder Bornylacetat unnötig, da die Filmbildung durch das Johanniskrautöl durch einfaches Verteilen der Zahnpasta auf den Zähnen und dem Zahnfleisch bereits bewirkt wird und die Schutzfunktion unmittelbar eintritt. Da es durchaus umstritten ist, ob das häufig empfohlene, langanhaltende Bürsten der Zähne günstig ist, liegt hier ein weiterer Vorteil der Erfindung, da die grobe mechanische Behandlung durchaus auch Schädigungen am Zahnfleisch hervorrufen kann. Weiterhin stellt sich sehr schnell ein Sauberkeitsgefühl ein, wodurch die Zahnpasta unter Verwendung der erfindungsgemäßen Bestandteile als sehr angenehm empfunden wird, und somit ein Anreiz zu ihrer Verwendung besteht. Der besonders hervorzuhebende Vorteil der Verwendung der erfindungsgemäßen Zahnpasta liegt jedoch, wie bereits ausgeführt, darin, daß durch die Bildung eines Schutzfilms, der entzündungshemmende Substanzen enthält, eine karies- und parodontosehemmende Wirkung auch dann eintritt, wenn die Zähne nur ein- bis zweimal, insbesondere aber nicht nach jeder kleineren Mahlzeit, gereinigt werden.

Unter den zahlreichen möglichen galenischen Formulierungen von Zahnpasten erweist sich im Zusammenhang mit den erfindungsgemäßen Wirksubstanzen eine Zusammensetzung der Zahnpasta, die neben den genannten Bestandteilen die üblichen Schleifmittel, Binde- und Verdickungsmittel, kapillaraktive Substanzen sowie Aromastoffe enthält. Um eine besonders wirksame Verteilung der Zahnpasta in der Mundhöhle und somit eine besonders gute Schutzfilmbildung zu erreichen, können übliche Substanzen, beispielsweise Tenside verwendet werden, die eine besonders gleichmäßige Verteilung der erfindungsgemäßen Substanzen gewährleisten.

Durch die geschilderte Wirkung des Johanniskrautöls, sich in kürzester Zeit als schützender Überzug auf Zähne und Zahnfleisch zu legen, eignet sich das erfindungsgemäße Mittel zur Mundpflege ganz besonders als Mundwasser. Bereits durch ein kurzes Spülen der Mundhöhle wird die besonders vorteilhafte Wirkung erzielt.

Dem Mundwasser können neben den genannten Bestandteilen desinfizierende und kapillaraktive Substanzen sowie Aromastoffe zugeführt werden. Anders als bei bekannten Mundwässern, die üblicherweise zum Beseitigen oder Überdecken von Mundgeruch verwendet werden, wird hier ein Mundwasser zur Verfügung gestellt, daß zur Prophylaxe und Therapie von Karies und Parodontose geeignet ist.

Die Erfindung betrifft auch die Verwendung von Johanniskrautöl mit Brenztraubensäure und L-Borneol und/oder Bornylacetat zur Herstellung eines Mundpflegemittels zur Prophylaxe von Karies und Parodontose durch Filmbildung. Das Neue, Erfinderische und Vorteilhafte dieser Verwendung und der Verwendung gemäß Ansprüchen 9 bis 14 wurde oben bei der Diskussion über die Vorteile des neuen und erfinderischen Mittels zur Mundpflege ausführlich dargelegt.

Die Erfindung wird an Hand von Beispielen näher erläutert.

Beispiel I

Unter Verwendung der im nachstehenden genannten Substanzen wird auf übliche Weise eine Zahnpasta hergestellt:

| | | |
|---|---|---|
| Glycerin 86%ig | 10,0 | g |
| Dicalciumphosphat wasserfrei | 20,0 | g |
| Titandioxid (Weißmacher) | 1,0 | g |
| Kolloidale Kieselsäure 35–40 µm, estra trocken | 1,0 | g |
| Natriumlaurylsulfat | 2,5 | g |
| Calciumcarbonat praec. | 22,0 | g |
| Methylcellulose (Füllstoff, Viskosmacher) | 1,0 | g |
| Aromastoff (versch. Öle) | 1,8 | g |
| Saccharin-Na | 0,15 | g |
| Johanniskrautöl | 7,0 | g |
| Brenztraubensäure | 0,1 | g |
| L-Borneol | 0,1 | g |
| Wasser | 33,35 | g |
| | 100,00 | g |

Eine auf diese Weise hergestellte Zahnpasta vermittelt unmittelbar nach ihrer Verwendung ein angenehmes Sauberkeitsgefühl und wirkt durch die Bildung eines Schutzfilms mit entzündungshemmender Komponente den Volkskrankheiten Karies und Parodontose wirkungsvoll und nachhaltig entgegen.

Beispiel II

Unter Verwendung der nachstehend genannten Substanzen wird auf übliche Weise ein Mundwasser hergestellt, wobei bei der Herstellung darauf zu achten ist, daß das Mundwasser in Form einer Emulsion vorliegt, damit die filmbildende Wirkung erhalten bleibt. Insbesondere sollten daher hochtourige Rührer verwendet werden.

| | | |
|---|---|---|
| Äthylalkohol 40%ig | 10 | ml |
| Aromastoffe (versch. Öle) | 3 | ml |
| Johanniskrautöl | 5 | ml |
| Brenztraubensäure | 0,1 | ml |
| L-Borneol | 0,1 | ml |
| Wasser | 81,8 | ml |
| | 100,00 | ml |

**Patentansprüche**

1. Mittel zur Mundpflege, insbesondere in Form von Zahnpasta oder Mundwasser, enthaltend Johanniskrautöl, dadurch gekennzeichnet, daß es zusätzlich Brenztraubensäure und L-Borneol und/oder Bornylacetat enthält.

2. Mittel nach Anspruch 1, dadurch gekennzeichnet, daß das Johanniskrautöl in einem Anteil von 5 bis 15 Gew.-%, vorzugsweise 7 Gew.-%, enthalten ist.

3. Mittel nach Anspruch 1 und/oder 2, dadurch gekennzeichnet, daß das Verhältnis von Johanniskrautöl zu Brenztraubensäure 70 : 1 bis 1000 : 1, vorzugsweise 100 : 1, und das Verhältnis von Johanniskrautöl zu L-Borneol und/oder Bornylacetat 70 : 1 bis 1000 : 1, vorzugsweise 100 : 1, beträgt.

4. Mittel nach mindestens einem der vorhergehenden Ansprüche, dadurch gekennzeichnet, daß es in Form einer Zahnpasta vorliegt.

5. Mittel nach Anspruch 4, dadurch gekennzeichnet, daß die Zahnpasta neben den genannten Bestandteilen Schleifmittel, Binde- und Verdickungsmittel, Kapillar-aktive Substanzen sowie Aromastoffe enthält.

6. Mittel nach mindestens einem der Ansprüche 1 bis 3, dadurch gekennzeichnet, daß es in Form eines Mundwassers vorliegt.

7. Mittel nach Anspruch 6, dadurch gekennzeichnet, daß das Mundwasser neben den genannten Bestandteilen desinfizierende sowie Kapillar-aktive Substanzen sowie Aromastoffe enthält.

8. Verwendung von Johanniskrautöl, Brenztraubensäure und L-Borneol und/oder Bornylacetat zur Herstellung eines Mundpflegemittels, das zur Prophylaxe von Karies und Parodontose durch Filmbildung geeignet ist.

9. Verwendung nach Anspruch 8, dadurch gekennzeichnet, daß das Johanniskrautöl in dem Mundpflegemittel in einem Anteil von 5 bis 15 Gew.-%, vorzugsweise 7 Gew.-%, enthalten ist.

10. Verwendung nach Anspruch 9, dadurch gekennzeichnet, daß das Verhältnis von Johanniskrautöl zu Brenztraubensäure 70 bis 100 : 1, vorzugsweise 100 : 1 und das Verhältnis von Johanniskrautöl zu L-Borneol und/oder Bornylacetat 70 bis 1000 : 1, vorzugsweise 100 : 1, beträgt.

11. Verwendung nach mindestens einem der Ansprüche 8 bis 10, dadurch gekennzeichnet, daß das Mundpflegemittel in Form einer Zahnpasta vorliegt.

12. Verwendung nach Anspruch 11, dadurch gekennzeichnet, daß die Zahnpasta neben den genannten Bestandteilen Schleifmittel, Binde- und Verdickungsmittel, Kapillar-aktive Substanzen sowie Aromastoffe enthält.

13. Verwendung nach mindestens einem der Ansprüche 8 bis 10, dadurch gekennzeichnet, daß das Mundpflegemittel in Form eines Mundwassers vorliegt.

14. Verwendung nach Anspruch 13, dadurch gekennzeichnet, daß das Mundwasser neben den genannten Bestandteilen desinfizierende und Kapillar-aktive Substanzen und Aromastoffe enthält.

## Claims

1. Oral hygiene product, especially in the form of toothpaste or mouthwash, containing St. John's wort oil, characterized in that it additionally contains pyruvic acid and L-borneol and/or bornyl acetate.

2. Product according to Claim 1, characterized in that the St. John's wort oil is contained in a proportion of 5 to 15% by weight, preferably 7% by weight.

3. Product according to Claim 1 and/or 2, characterized in that the ratio of St. John's wort oil to pyruvic acid is 70 : 1 to 1000 : 1, preferably 100 : 1, and the ratio of St. John's wort oil to L-borneol and/or bornyl acetate is 70 : 1 to 1000 : 1, preferably 100 : 1.

4. Product according to at least one of the preceding claims, characterized in that it is present in the form of a toothpaste.

5. Product according to Claim 4, characterized in that, in addition to the said components, the toothpaste contains abrasives, binding agents and thickeners, capillary-active substances as well as flavourings.

6. Product according to at least one of Claims 1 to 3, characterized in that it is present in the form of a mouthwash.

7. Product according to Claim 6, characterized in that, in addition to the said components, the mouthwash contains disinfectant as well as capillary-active substances as well as flavourings.

8. Use of St. John's wort oil, pyruvic acid and L-borneol and/or bornyl acetate for the production of an oral hygiene product which is suitable for the prophylaxis of caries and paradontosis by film formation.

9. Use according to Claim 8, characterized in that the St. John's wort oil is contained in the oral hygiene product in a proportion of 5 to 15% by weight, preferably 7% by weight.

10. Use according to Claim 9, characterized in that the ratio of St. John's wort oil to pyruvic acid is 70 to 100 : 1, preferably 100 : 1, and the ratio of St. John's wort oil to L-borneol and/or bornyl acetate is 70 to 1000 : 1, preferably 100 : 1.

11. Use according to at least one of Claims 8 to 10, characterized in that the oral hygiene product is present in the form of a toothpaste.

12. Use according to Claim 11, characterized in that, in addition to the said components, the toothpaste contains abrasives, binding agents and thickeners, capillary-active substances as well as flavourings.

13. Use according to at least one of Claims 8 to 10, characterized in that the oral hygiene product is present in the form of a mouthwash.

14. Use according to Claim 13, characterized in that, in addition to the said components, the mouthwash contains disinfectant and capillary-active substances and flavourings.

## Revendications

1. Produit pour soins de la bouche, en particulier sous forme d'une pâte dentifrice ou d'une eau dentifrice, contenant de l'essence de millepertuis et caractérisé en ce qu'il contient en outre de l'acide pyruvique et du L-bornéol et/ou de l'acétate de bornyle.

2. Produit selon la revendication 1, caractérisé en ce que l'essence de millepertuis est contenue en une proportion de 5 à 15% en poids, avantageusement 7% en poids.

3. Produit selon la revendication 1 et/ou 2, caractérisé en ce que le rapport de l'essence de millepertuis à l'acide pyruvique va de 70 : 1 à 1000 : 1, et est avantageusement égal à 100 : 1, et en ce que le rapport de l'essence de millepertuis au L-bornéol et/ou à l'acétate de bornyle va de 70 : 1 à 1000 : 1 et est avantageusement égal à 100 : 1.

4. Produit selon au moins l'une des revendications précédentes, caractérisé en ce qu'il est sous forme d'une pâte dentifrice.

5. Produit selon la revendication 4, caractérisé en ce que la pâte dentifrice contient, en plus des constituants cités, un produit pour polissage, du liant et de l'épaississant, des substances actives sur les capillaires ainsi que des substances aromatiques.

6. Produit selon au moins l'une des revendications 1 à 3, caractérisé en ce qu'il est sous forme d'une eau dentifrice.

7. Produit selon la revendication 6, caractérisé en ce que l'eau dentifrice contient, en plus des constituants précités, des substances désinfectantes ainsi que des substances actives sur les capillaires et des substances aromatiques.

8. Utilisation de l'essence de millepertuis, de l'acide pyruvique et du L-bornéol et/ou de l'acétate de bornyle pour préparer un produit pour les soins de la bouche, qui convient, par formation d'une pellicule, pour la prophylaxie d'une carie et de la parodontose.

9. Utilisation selon la revendication 8, caractérisée en ce que l'essence de millepertuis est contenue dans le produit pour soins de la bouche en une proportion de 5 à 15% en poids, avantageusement de 7% en poids.

10. Utilisation selon la revendication 9, caractérisée en ce que le rapport entre l'essence de millepertuis et l'acide pyruvique est de 70 à 100 : 1, avantageusement égal à 100 : 1, et en ce que le rapport de l'essence de millepertuis au L-bornéol et/ou à l'acétate de bornyle va de 70 à 1000 : 1 et est avantageusement égal à 100 : 1.

11. Utilisation selon au moins l'une des revendications 8 à 10, caractérisée en ce que le produit pour les soins de la bouche est sous la forme d'une pâte dentifrice.

12. Utilisation selon la revendication 11, caractérisée en ce que la pâte dentifrice contient, en plus des constituants cités, un produit pour polissage, un liant et un épaississant, des substances actives sur les capillaires ainsi que des substances aromatiques.

13. Utilisation selon au moins l'une des revendications 8 à 10, caractérisée en ce que le produit pour les soins de la bouche est sous la forme d'une eau dentifrice.

14. Utilisation selon la revendication 13, caractérisée en ce que l'eau dentifrice contient, en plus des constituants cités, des substances désinfectantes et des substances actives sur les capillaires ainsi que des substances aromatiques.